(19) Europäisches Patentamt
European Patent Office
Office européen des brevets

(11) **EP 4 000 515 A1**

(12) **EUROPEAN PATENT APPLICATION**

(43) Date of publication:
**25.05.2022 Bulletin 2022/21**

(21) Application number: **20209475.1**

(22) Date of filing: **24.11.2020**

(51) International Patent Classification (IPC):
*A61B 5/145* (2006.01)    *A61B 5/00* (2006.01)

(52) Cooperative Patent Classification (CPC):
**A61B 5/4266; A61B 5/14517; A61B 5/6833;**
A61B 2560/0276

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **DELLIMORE, Kiran Hamilton J.**
  **5656 AE Eindhoven (NL)**
• **HUIJBREGTS, Laurentia Johanna**
  **5656 AE Eindhoven (NL)**
• **JOHNSON, Mark Thomas**
  **5656 AE Eindhoven (NL)**

(74) Representative: **Philips Intellectual Property &
Standards
High Tech Campus 52
5656 AG Eindhoven (NL)**

(54) **SYSTEMS AND METHODS FOR INDICATING A NEED TO REPLACE A WEARABLE PATCH**

(57)    A wearable patch system comprises a sensor (14) for measuring at least one sweat parameter, and a controller (16) for indicating the need to replace the patch. The controller is configured to receive the at least one sweat parameter and compare the at least one sweat parameter to a first threshold. The controller is also configured to determine the replacement information based on the total amount of time during which the at least one sweat parameter exceeds the first threshold; and/or a value indicative of the at least one sweat parameter in respect of the total amount of time during which the at least one sweat parameter exceeds the first threshold. The controller is further configured to output an indication that the wearable patch needs (12) replacing based on the replacement information.

FIG. 2

EP 4 000 515 A1

**Description**

FIELD OF THE INVENTION

This invention relates to wearable patches for sensing physiological parameters. In particular, the invention relates to determining when a wearable patch needs to be replaced.

BACKGROUND OF THE INVENTION

Patches used in the monitoring of patients in clinical settings may require replacement, earlier than their intended usage lifetime, for many reasons. These may include skin irritation which may arise from poor material biocompatibility or due to low air/water-permeability or due to build-up of sweat trapped between the patch and the skin. Poor patch-to-skin coupling may arise due to weakening of the adhesive caused by moisture from sweating or ablution which can lead to unintended repositioning of the patch, i.e., shifting and/or rotation, which may have adverse consequences on measurement quality and calibration. Depletion of reagents used in biomarker analysis, and dehydration of conductive gels used to lower contact impedance between the patch electrode and the skin may also arise, leading to deterioration in signal quality. Degradation of the battery powering the sensors on the patch may also arise as a result of corrosion caused by the interaction of sodium chloride in sweat with the metal in the batteries or acid corrosion caused by a drop in the pH of the sweat.

A key unmet challenge remains the determination of the optimal replacement moment for monitoring patches, which varies from patient to patient (due to factors such as sweating level which is influenced by age, gender and medication, e.g., cholinesterase inhibitors, selective serotonin reuptake inhibitors, opioids and tricyclic antidepressants). This optimal replacement moment also depends heavily on environmental factors such as humidity and ambient temperature. Monitoring patches in hospitals are often replaced on a fixed schedule, e.g., during daily during nurse rounds, or on an *ad hoc* basis when patches fail unexpectedly. In both cases, current patch replacement strategies are suboptimal since in the former case well-functioning patches may be unnecessarily replaced which is time consuming for nurses and costly, and in the latter case patch failure may occur at critical moments which may compromise patient safety by leaving the patient without adequate monitoring over a period of time or by triggering excessive false alarms which contribute to nurse alarm fatigue, which can lead to non-response to critical alarms.

US 2017/0056650 A1 describes electrode patches for medical devices comprising means to indicate the end of life of a patch. One way of determining the end of life of a patch thereby involves measuring the overall exposure of the patch to sweat.

This device may provide an improvement over a patch replacement on a fixed schedule or upon failure.

SUMMARY OF THE INVENTION

It has been shown though that the use of the overall exposure to sweat as an end of life indicator can still lead to patches being replaced significantly earlier than necessary. There remains a need for systems and methods that can help to determine the optimal time for the replacement of a wearable patch.

The invention is defined by the independent claims. The dependent claims define advantageous embodiments.

According to examples in accordance with an aspect of the invention, there is provided a wearable patch system, comprising:

a wearable patch comprising a sensor for measuring at least one sweat parameter; and
a controller for indicating the need to replace the patch,
wherein the controller is configured to:

receive the at least one sweat parameter;
compare the at least one sweat parameter to a first threshold;
determine replacement information based on:

the total amount of time during which the at least one sweat parameter exceeds the first threshold; and/or
a value indicative of the at least one sweat parameter in respect of the total amount of time during which the at least one sweat parameter exceeds the first threshold; and

output an indication that the wearable patch needs replacing based on the replacement information.

Patches used in the monitoring of patients in clinical settings may, for many reasons, require replacement earlier than their intended usage lifetime. These reasons may include poor patch-to-skin coupling due to weakening of the adhesive caused by moisture from sweating or ablution which can lead to unintended repositioning of the patch, i.e., shifting and/or rotation, which may have adverse consequences on measurement quality and calibration, depletion of reagents used in biomarker analysis, dehydration of conductive gels used to lower contact impedance between the patch electrode and the skin leading to deterioration in signal quality, or due to degradation of the battery powering the sensors on the patch as a result of corrosion caused by the interaction of sodium chloride in sweat with the metal in the batteries or acid corrosion caused by a drop in the pH of the sweat.

The optimum time for replacing the patch is thereby often not simply a function of the overall amount of sweat excreted by a patient or the overall amount of particular contents of the sweat excreted by a patient. To the contrary, most patches are designed to withstand a certain baseline amount of sweat. The amount of sweat excreted by a patient and its composition though is rarely constant and can vary greatly over time. Spikes in the amount of sweat excreted or in one or more components of the excreted sweat can significantly impact their useful lifetime.

By determining the amount of time a patch is exposed to volumes of sweat and/or amounts of given components in the sweat in excess of a threshold, a more precise prediction can be made on the optimum timing for the replacement of the patch. In particular, determining the total amount of sweat excreted or the total amount of given components in the sweat in those periods of time when the patch is exposed to volumes of sweat and/or amounts of the given components in the sweat in excess of a threshold can enable a more precise prediction of the optimum timing for the replacement of the patch. In particular, it is possible to avoid a needlessly early replacement of the patch.

The replacement information can, for example, comprise information that the total amount of time during which the at least one sweat parameter exceeds the first threshold exceeds a time threshold and/or that the value indicative of the at least one sweat parameter in respect of the total amount of time during which the at least one sweat parameter exceeds the first threshold exceeds a parameter threshold.

The value indicative of the at least one sweat parameter in respect of the total amount of time may comprise an integration over time of a measured amount of a sweat component excreted over said total amount of time or the rate of excretion over said total amount of time.

In addition, the patch may further comprise at least one further sensor for monitoring a further physiological parameter of the wearer.

The patch can, for example, have a primary function of monitoring the further physiological parameter, and the sweat sensor is used primarily for determining the need for patch replacement.

The at least one further sensor can be selected from sensors for monitoring vital signs, sensors for biomarkers and combinations thereof.

While the system in its simplest form can be used to determine the optimum replacement time for a sensor for a sweat parameter it is particularly useful to determine the optimum replacement time of patches including further sensors.

Furthermore, the at least one sweat parameter may be selected from sweat volume, sweat pH, amount or rate of excretion of sodium, amount or rate of excretion of potassium, amount or rate of excretion of chloride, the amount or rate of excretion of at least one biomarker present in sweat and combinations thereof.

The above parameters can have a direct impact on the lifetime of a wearable patch. Accordingly, the use of these parameters as the sweat parameter provides particularly precise information on the optimum timing for the replacement of the patch.

In addition, the sweat sensor may measure at least two sweat parameters.

Measuring multiple sweat parameters makes it possible to take into account multiple different failure modes of the patch caused by e.g. by different components of the sweat such as the failure of a battery or the failure of the adhesive attaching the patch to the wearers body. This further improves the accuracy of the determination of the optimum timing for the replacement of the patch.

The system may further comprise:

an output device for delivering the indication to a user of the system; and/or
a communications system for communicating with an external output device for delivering the indication to a user of the system.

In particular, in clinical settings it can be advantageous if the indication that the wearable patch needs to be replaced is transmitted to e.g. a central monitoring station or a device carried by a nurse responsible for a patient to ensure that the information that a patch needs replacing is received in a timely manner by a person responsible for the replacement of the patch.

In the case of outpatients, it can be useful to provide an indication that the patch needs replacing directly to the wearer so that the wearer can replace it him/herself or inform their doctor or nurse that the patch needs replacing.

The communications system may be a wireless communications system.

The use of wireless communications is particularly convenient in a clinical setting where nurses regularly move around the wards.

According to examples in accordance with a further aspect of the invention, a method for providing an indication of the need to replace a wearable patch is provided. The method comprises the steps of:

measuring at least one sweat parameter of a person wearing the patch;
comparing the at least one sweat parameter with a first threshold;
determining replacement information based on:

the total amount of time during which the at least one sweat parameter exceeds the first threshold; and/or
a value indicative of the at least one sweat parameter in respect of the total amount of time during which the at least one sweat parameter exceeds the first threshold; and

providing an indication that the wearable patch needs replacing based on the replacement information.

The replacement information may comprise information whether the total amount of time during which the at least one sweat parameter exceeds the first threshold exceeds a time threshold and/or the value indicative of the at least one sweat parameter in respect of the total amount of time during which the at least one sweat parameter exceeds the first threshold exceeds a value threshold.

The present method can provide more accurate information on when a patch worn by a person needs to be replaced. In particular, the method can be used to avoid a needlessly early replacement of the patch.

In addition, the at least one sweat parameter may be selected from sweat volume, sweat pH, amount or rate of excretion of sodium, amount or rate of excretion of potassium, amount or rate of excretion of chloride, the amount or rate of excretion of at least one biomarker present in sweat and combinations thereof.

The above parameters can have a direct impact on the lifetime of a wearable patch. Accordingly, the use of these parameters as the sweat parameter provide particularly precise information on the optimum timing for the replacement of the patch.

Furthermore, the step of measuring at least one sweat parameter may comprise measuring at least two sweat parameters.

Measuring multiple sweat parameters makes it possible to take into account multiple different failure modes of the patch caused by e.g. by different components of the sweat such as the failure of a battery or the failure of the adhesive attaching the patch to the wearers body. This further improves the accuracy of the determination of the optimum timing for the replacement of the patch.

The value indicative of the at least one sweat parameter in respect of the total amount of time may comprise an integration over time of a measured amount of a sweat component excreted over said total amount of time or the rate of excretion over said total amount of time.

According to examples in accordance with a further aspect of the invention, there is provided computer program comprising computer program code which is adapted, when said program is run on a computer, to implement the above method.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a first example of a wearable patch system according to the invention;
Fig. 2 shows a graph tracing the amount of sodium excreted by a subject and the performance of a wearable patch over time;
Fig. 3 shows a graph tracing the amount of sodium as well as the volume of sweat excreted by a subject and the performance of a wearable patch over time;
Fig. 4 shows a second example of a wearable patch system according to the invention; and
Fig. 5 shows a flow diagram for an example of the method for providing an indication of the need to replace a wearable patch of the invention.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for indicating the need to replace a patch. The system comprises a wearable patch comprising a sensor for measuring at least one sweat parameter and a controller for indicating the need to replace the patch. The controller is configured to receive the at least one sweat parameter and to compare the at least one sweat parameter to a first threshold. The controller is further configured to determine replacement information based on the total amount of time during which the at least one sweat parameter exceeds the first threshold and/or a value indicative of the at least one sweat parameter in respect of the total amount of time during which the at least one sweat parameter exceeds the first threshold and to output an indication that the wearable patch needs replacing based on the replacement information.

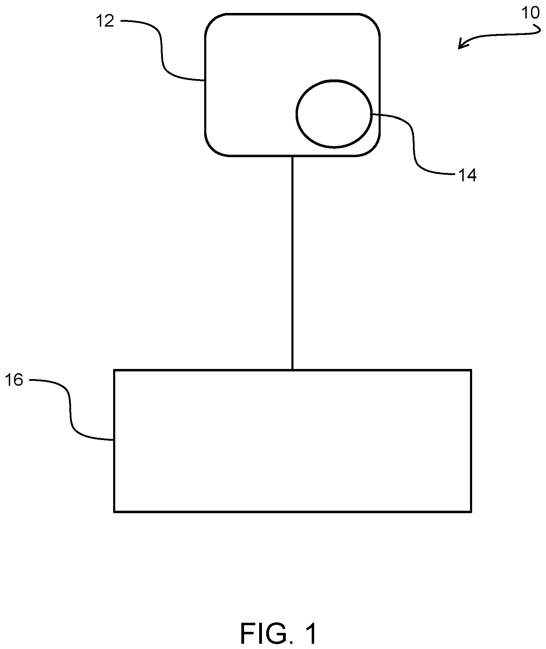

Fig. 1 shows an example of a wearable patch system 10 of the invention. The system 10 comprises a wearable patch 12 comprising a sensor 14 for measuring at least one sweat parameter. The system further comprises a controller 16.

The wearable patch 12 can be any form of wearable patch. In its most basic form the patch is a patch for measuring at least one sweat parameter. In other instances, the wearable patch 12 can be a patch for measuring other parameters such as vital signs of the wearer or biomarkers excreted by the wearer. The wearable patch 12 may also fulfil further functions such as drug delivery.

The sensor 14 can e.g. be any sensor that is capable of sensing a parameter indicative of the sweat rate of a person. Examples of sensors that can be employed as the sensor 14 include galvanic skin response (GSR) sensors, calorimetric flow sensors, microbalances or resonance based sensors. MEMS calorimetric flow sensors measure the flow rate by measuring the asymmetry of the temperature profile modulated by the flow of sweat through e.g., a microchannel. Microbalances/resonance based sensors (e.g., a quartz crystal microbalance) measure a mass variation per unit area by measuring the change in frequency of a quartz crystal resonator. Other sensors include sensors for biomarkers that are indicative of the amount of sweat excreted by a person such as sodium ions, potassium ions or lactate. The sensor can, for example work based on electrochemical measurements.

The sensor 14 is in communication with the controller 16. The communication can be wired or wireless, such as a Bluetooth or NFC connection.

The controller 16 receives the sweat parameter from the sensor 14. The controller 16 is configured to compare the at least one sweat parameter to a first threshold and determine a replacement information. The replacement information is determined based on the total amount of time during which the at least one sweat parameter exceeds the first threshold and/or a value indicative of the at least one sweat parameter in respect of the total amount of time during which the at least one sweat parameter exceeds the first threshold. The controller 16 is further configured to output an indication that the wearable patch 12 needs replacing based on the replacement information.

The at least one sweat parameter may be selected from sweat volume, sweat pH, amount or rate of excretion of sodium, amount or rate of excretion of potassium, amount or rate of excretion of chloride, the amount or rate of excretion of at least one biomarker present in sweat and combinations thereof.

In general, patch lifetime is logically assumed to be longer at lower sweat rates, lower sweat volumes and lower exposure to $Na^+$ or $K^+$ ions. This further implies that conditions of lower humidity and temperature, in which sweat rates are typically lower will also be linked to longer patch lifetimes. Similar logic applies with respect to reagent depletion, with more reagent consumed when detecting biomarkers at higher sweat rates and volumes.

Hence, it has in the past been assumed that patch replacement due to performance degradation is determined by the overall value of a given sweat parameter. It has thus been assumed that patch failure would occur once the overall value of the respective sweat parameter has exceeded a predestined threshold.

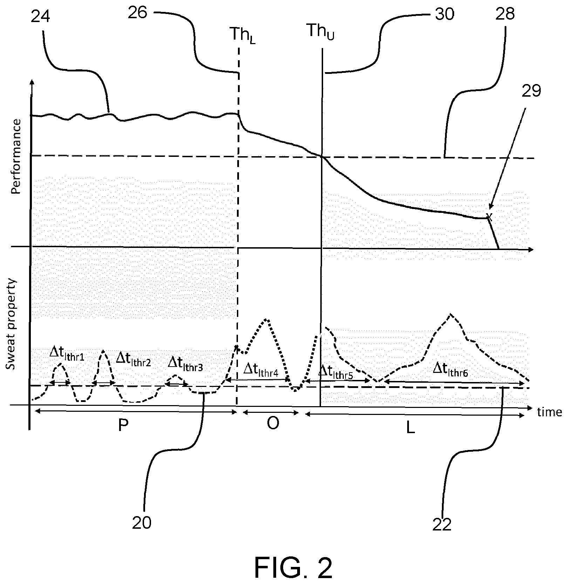

However, in some instances the sweat parameter varies non-monotonically over time. Such a situation is shown by the graph in Fig. 2. This may occur, for example, in the case of sweat pH or the amount of $Na^+$ an $K^+$ ions excreted (as measured for example by impedance) but also in case of the sweat volume itself which may rise and fall over time. Furthermore, it has been recognised that an exposure to sweat or one or more of the components of sweat under a threshold that is specific to the type of patch, does not impact the lifetime of a patch in a significant way.

In Fig. 2 graph 20 shows the development of the amount of sodium excreted by the wearer of a patch over time in an exemplary fashion. It is clear from Fig. 2 that the amount of sodium excreted varies non-monotonically over time. For some of the time the amount of sodium is below a first threshold 22 and at other points in time the first threshold 22 is exceed. Furthermore, graph 24 in Fig. 2 shows the development of the performance of a patch over time. In the beginning the performance is stable and there is no need to replace the patch. This is a time period "P" when it is premature to replace the patch. After the patch is exposed to sodium excreted by the wearer for some time the performance begins to deteriorate. In this case the point where the performance of the patch starts to deteriorate is defined as the lower threshold 26 ($Th_L$). This lower threshold 26 marks the earliest point in time where it is sensible to replace the patch. After further deterioration a point is reached where the performance of the patch drops below the minimum acceptable performance level 28. This point is defined as the upper threshold 30 ($Th_U$). The upper threshold 30 marks the latest point where the patch should be replaced in order to avoid an unacceptable loss of performance and in an extreme case the catastrophic failure of the patch. There is a time period O between these time points which represents the optimum time for patch replacement. The lower threshold 26 and the upper threshold 30 thereby define the optimum replacement window for the patch.

Catastrophic failure of the patch is represented at point 29.

In cases such as the case shown in Fig. 2 a simple thresholding approach will be inadequate for determination of the optimal replacement time window. Instead, a total amount of time spent above a first threshold, $T_{tot,thr}$, may be used to provide a more reliable estimation of the failure condition, where, $T_{tot,thr} = \sum_{i=1}^{n} \Delta t_{thr,i}$. Exemplary values for this first threshold could be a sweat rate of 2.5 nl/min/gland or a rate of the excretion of sodium of 35 mmol/h.

$\Delta t_{thr,i}$ is an $i^{th}$ time duration during which the first threshold 22 of the sweat property is exceeded. The sum of all of these time durations gives a total time period during which the threshold 22 is exceeded.

The need to replace the patch can thereby be determined based on a ratio of maximum allowable time above a time threshold, $T_{max}$, as follows:

$$\text{if } T_{tot,thr} / T_{max} \geq 1.0 \quad \rightarrow \text{imminent catastrophic failure} \qquad (1)$$

$$\text{if } 0.9 \leq T_{tot,thr} / T_{max} < 1 \quad \rightarrow \text{failure warning} \qquad (2)$$

$$\text{if } 0.75 \leq T_{tot,thr} / T_{max} < 0.9 \quad \rightarrow \text{optimal replacement window} \qquad (3)$$

$$\text{if } T_{tot,thr} / T_{max} < 0.75 \quad \rightarrow \text{premature replacement} \qquad (4)$$

Here $T_{max}$ may be determined empirically or based on manufacturing test standards.

An example for such a situation can be an individual engaging in a physical activity of variable intensity which induces them to excrete sweat at a variable rate, with a $Na^+$ ion concentration that is also variable. In such a situation the sweat rate and the rate of sodium excretion is high (above the first threshold) for certain periods and for other periods it is low (below the first threshold). For instance, such an activity could be interval running or powerlifting, or even just walking up a flight of stairs and then walking on level ground. If it is assumed that the individual spends 15 mins in low intensity physical activity in which the sweat rate is below the first threshold of 2.5 nl/min/gland and/or the rate of sodium excretion is below 35 mmol/h (i.e., the first threshold for the sweat $Na^+$-ion concentration); and 30 mins in high intensity physical activity in which the sweat rate exceeds 2.5 nl/min/gland and/or the rate of sodium excretion exceeds 35mmol/L. The total time allowed above either threshold before replacement is needed in this case may be $T_{max}$ = 4 hours. In this situation the patch should ideally be replaced when the individual spends more than 3h and less than 3h and 30 minutes above either threshold.

Of course, other thresholds may be applied between the different conclusions, and there may be fewer conclusions, for example the failure warning and imminent failure may be grouped as a single failure indication.

The same approach can also be applied if a value indicative of the at least one sweat parameter in respect to the total amount of time during which the at least one sweat parameter exceeds the first threshold is used to determine the optimal replacement window for the patch. In this case a value threshold analogous to $T_{max}$ is used to determine the optimum replacement window.

The value indicative of the at least one sweat parameter in respect of the total amount of time may comprise an integration over time of a measured amount of a sweat component excreted over said total amount of time or the rate of excretion over said total amount of time. Thus for the time periods during which the first threshold 22 is exceeded, an integral of the sweat parameter is obtained (i.e. the area under the graph 20 for the portions above the threshold 22). This may be considered to be a value indicative of the sweat parameter in respect to the total amount of time during which the at least one sweat parameter exceeds the first threshold.

Preferably, a time threshold and/or the value threshold may be selected to ensure patch replacement prior to the occurrence of deterioration of patch performance (i.e., degradation in signal quality, complete reagent depletion or battery failure, etc.) and catastrophic failure. In practice, depending on the circumstance, the optimal replacement window may be on the order of at least tens of minutes to many hours.

As demonstrated above, the replacement information may comprise information that the total amount of time during which the at least one sweat parameter exceeds the first threshold exceeds a time threshold and/or that the value indicative of the at least one sweat parameter in respect of the total amount of time during which the at least one sweat parameter exceeds the first threshold exceeds a parameter threshold.

The parameter threshold may for example a value indicative of the overall amount of sodium excreted during the time the sodium excretion exceeds the first threshold.

In some instances, the system measures at least two sweat parameters.

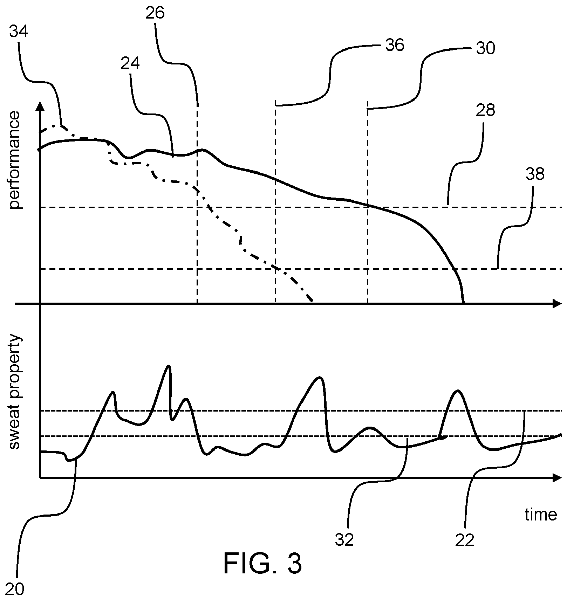

An exemplary scenario where the system measures at least two sweat parameters and the optimum replacement window is determined based on two performance parameters of the patch is shown in Fig. 3.

Fig. 3 again shows a graph 20 charting the development of a non-monotonic sweat parameter, in this case the volume of sweat excreted over time. Furthermore, Fig. 3 also shows a first sweat parameter threshold 22 for the sweat volume. This sweat parameter threshold 22 is a threshold impacting a first patch performance parameter as indicated in Fig. 3 by graph 24.

In addition, Fig. 3 also shows a second sweat parameter threshold 32. This second sweat parameter threshold 32 is a threshold impacting a second patch performance parameter as indicated in Fig. 3 by graph 34.

Fig. 3 further shows a first lower threshold 26, indicating the point where the performance of the first patch performance parameter begins to deteriorate, and a first upper threshold 30 where the first patch performance parameter drops below its minimum acceptable performance level 28 in analogy to the lower threshold 26 and upper threshold 28 of Fig. 2. In addition, Fig. 3 also shows a second upper threshold 36, indicating the point where the second patch performance parameter drops below its minimum acceptable performance level 38.

In the situation shown in Fig. 3, the optimum patch replacement window is between the first lower threshold 26 and the second upper threshold 36.

In this case, the optimal patch replacement window is thereby determined for a failure condition involving two or more performance parameters, i.e., a compound failure condition. In this case the lower and upper thresholds utilized may be adapted dynamically to circumvent catastrophic failure of all patch performance parameters. This will generally lead to a shortening of the optimal replacement time window as depicted in Fig. 3, for the exemplary case of two performance parameters. In this case two or more values for the first threshold, the time threshold and the value threshold may need to be defined. In practice depending on the circumstance, the optimal replacement window would still be on the order of at least tens of minutes to a few hours, which would give nurses adequate time to replace the patch at their convenience.

In case the system is such that both the first and the second patch performance parameters have a first and second lower threshold, it is preferable to define the replacement window as starting from the higher of the two lower thresholds in order to maintain optimal patch performance over the longest possible period of time. In case the system is such that both the first and the second patch performance parameters have a first and second lower threshold as well as a first and second upper threshold, it is preferable to define the replacement window as being between the higher of the lower thresholds and the lower of the upper thresholds as this maximizes the time of optimal patch performance while avoiding patch failure. The same approach can also be used in systems having more than two patch parameters.

The outputting of the indication that the wearable patch 12 needs replacing can take place by providing a signal directly to the user, for example by visual, aural or haptic means and/or by transmitting a signal to an external output device for example via a wired or wireless communication interface.

The indication of the need for patch replacement may, for example, be accomplished via both passive and active visual, aural or haptic means. In the case where the system comprises a communications system for communicating with an external output device, this may be via an alert on the user interface of a smartphone or other portable device wirelessly connected to the patch (active visual alert system). It is also possible for the patch to be connected to an external output device via a wired communications system. In the case where the system comprises an output device this may be via flashing LED lights (active visual alert system) or via a color change indicator triggered by a change in pH (passive visual alert system). Audio alerts may include a simple sound such as a beeping sound or it could be a spoken message. Haptic means can take the form of vibrating elements either internal or external to the patch. In practice it can preferable if the indication of the need for patch replacement during the optimal replacement window takes place in a complimentary fashion to the clinical workflow. Clinicians would ideally be alerted of the need for patch replacement at moments in which they are less occupied with clinical tasks, which would offer them sufficient time to find a suitable moment for patch replacement.

In addition to the failure of a wearable patch due to the exhaustion of reagents or the corrosion of electrodes, the system may also be used to alert a user to the fact that a patch has moved from its initial position. This may occur, for example, due to excessive sweating leading to a situation where an adhesive used to attach the patch to the skin of the user is no longer able to hold the patch in its initial and intended place.

The failure of the adhesive to keep the patch in its intended spot can lead to the migration and/or rotation of the patch and can have multiple consequences. Two exemplary measurements that are affected by such a migration and/or rotation of the patch are posture measurement patches and $SpO_2$ measurement patches.

The expression "posture measurement patch" thereby refers to a patch that measures for example whether a person wearing the device is upright (i.e. standing or sitting) or lying prone, supine, on his left side, on his right side, etc. This information per se is already very useful for the caretakers, for example for decubitus ulcer prevention. Furthermore, the information might also be used in algorithms that determine for example if the patient is ambulating or not.

In order for the posture measurement to be accurate, the orientation of the patch with respect to the body needs to be known. One way to achieve this is to always place the device in the same manner on the body, taking into account the body shape. Another way to achieve this is by calibrating the patch. If the sensor shifts or, even worse, rotates, the calibration is not valid anymore, because the determined direction of the patch with respect to the body axes has changed.

From the sweat measurements described above, it can be deduced whether a wearer has excreted so much sweat that it is likely that the patch has shifted/rotated. In this case, recalibration for posture and/or correction of the sweat rate discretization can be automatically triggered, or a nurse could be advised to remove/replace the patch. The latter could even be done in advance when it is predicted that the patch will soon be exposed to so much accumulated sweat that it will likely shift and/or rotate.

Optical blood oxygen saturation measurements (i.e. $SpO_2$ measurements) are delicate measurements, which involve empirical constants (which are often referred to as "calibration constants") that depend on the place on the body where the sensor is attached. Therefore, if a patch with an $SpO_2$ sensor starts to shift to another location or rotates, this might impact the calibration constants that should be used for the correct calculation of $SpO_2$. Therefore, a nurse should be advised when the patch has moved, to remove and replace the patch or to or enter new calibration constants according to the new position.

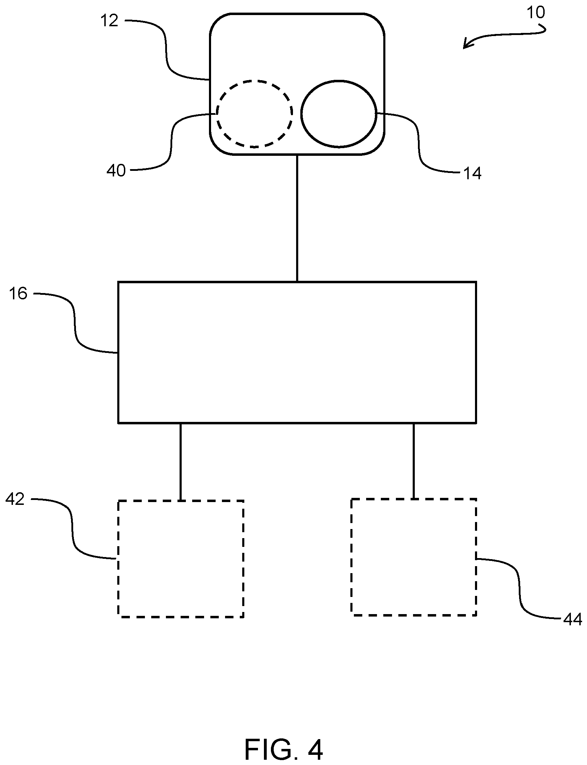

Fig. 4 shows a further example of a wearable patch system 10 of the invention. In analogy to the system shown in Fig. 1, the system 10 comprises a wearable patch 12 comprising a sensor 14 for measuring at least one sweat parameter and a controller 16.

The system further comprises a further sensor 40 for monitoring a further physiological parameter of the wearer. The at least one further sensor may be selected from sensors for monitoring vital signs and sensors for biomarkers. This further sensor can, for example, comprise one or more electrodes to monitor the heart function of a wearer, a sensor for a biomarker excreted by the body, a blood oxygen sensor, or a posture sensor for example an inertial sensor such as a gyroscope or an accelerometer. The system can thereby determine the optimum window for replacing the patch for example if electrode performance drops due to corrosion caused by exposure to sweat or if reagents used to sense biomarkers are depleted as they are eluted by sweat or consumed in the analysis of sweat.

The system also comprises an output device 42 for delivering the indication to a user of the system and/or a communications system 44 for communicating with an external output device for delivering the indication to a user of the system.

As described above, the indication that the patch needs to be replaced can either be communicated directly to the wearer or to another user, such as a nurse responsible for the wearer by any suitable means. The means include visual, aural or haptic means of conveying information. The means can be active, e.g. a flashing LED, a message to smartphone or table, a beeping sound or a vibration, or passive, e.g. via the color change of an indicator. In one example, the indication is transmitted to an external device such as a smartphone or tablet carried by a nurse. In this case the system can be designed such that a user is provided with a window in which the replacement should take place and can plan the replacement such that it does fits within the overall schedule, for example by replacing the patch during a scheduled visit or during less busy times. Alternatively, the means for providing the indication that the patch needs replacing can be integrated in the patch itself.

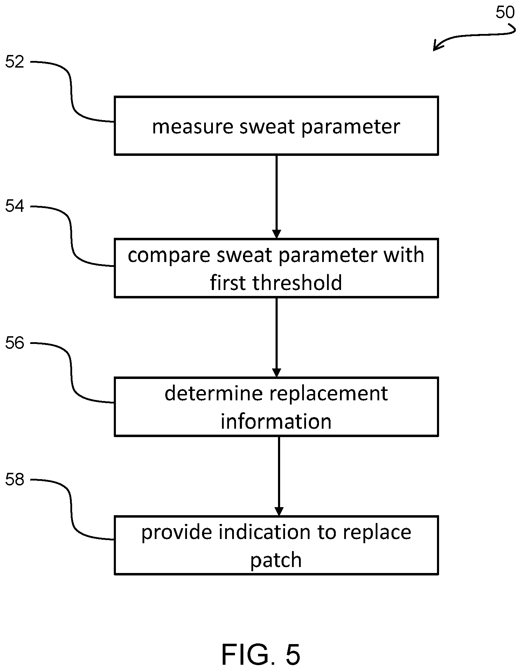

Fig. 5 shows an example of a method 50 for providing an indication of the need to replace a wearable patch according to the invention. The method comprises the steps of measuring 52 at least one sweat parameter of a person wearing the patch, comparing 54 the at least one sweat parameter with a first threshold, determining 56 replacement information based on the total amount of time during which the at least one sweat parameter exceeds the first threshold and/or a value indicative of the at least one sweat parameter in respect of the total amount of time during which the at least one sweat parameter exceeds the first threshold and providing 58 an indication that the wearable patch needs replacing based on the replacement information.

The replacement information may comprise information that the total amount of time during which the at least one sweat parameter exceeds the first threshold exceeds a time threshold and/or the value indicative of the at least one sweat parameter in respect of the total amount of time during which the at least one sweat parameter exceeds the first threshold exceeds a parameter threshold.

The method of Fig. 5 can provide more accurate information on when a patch worn by a person needs to be replaced. In particular, the method can be used to avoid a needlessly early replacement of the patch.

The at least one sweat parameter measured in the step of measuring 52 may be selected from sweat volume, sweat pH, amount or rate of excretion of sodium, amount or rate of excretion of potassium, amount or rate of excretion of chloride, the amount or rate of excretion of at least one biomarker present in sweat and combinations thereof.

Parameters such as the sweat volume, the sweat pH, the amount of certain ions excreted with the sweat or the amount of biomarkers excreted with the sweat can have a direct impact on the lifetime of a wearable patch. For example, some of the ions present in sweat can have corrosive effects on electrodes integrated in a patch. Furthermore, the amount of certain ions in the sweat can impact the skin conductivity and, hence, the drain on batteries integrated in a patch. The sweat pH and sweat volume can have an impact on adhesives used to attached a patch to the skin of the wearer. Biomarkers and sweat volume can impact the speed at which reactants present in a patch for analytical purposes are consumed or eluted. Measuring such parameters as the sweat parameter can therefore provide particularly precise information on the optimum timing for the replacement of the patch.

Furthermore, in some examples the step of measuring 52 at least one sweat parameter comprises measuring at least two sweat parameters.

Measuring multiple sweat parameters makes it possible to take into account multiple different failure modes of the patch caused by e.g. by different components of the sweat such as the failure of a battery or the failure of the adhesive attaching the patch to the wearers body. This further improves the accuracy of the determination of the optimum timing for the replacement of the patch.

In the method shown in Fig. 5, the value indicative of the at least one sweat parameter in respect of the total amount of time may comprise an integration over time of a measured amount of a sweat component over said total amount of time or the rate of excretion over said total amount of time.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. Measures are recited in mutually different dependent claims can be advantageously combined. If the term "adapted to" is used in the claims or description, it is noted the term "adapted to" is intended to be equivalent to the term "configured to". Any reference signs in the claims should not be construed as limiting the scope.

A computer program may be stored/distributed on a suitable medium, such as an optical storage medium or a solid-state medium supplied together with or as part of other hardware, but may also be distributed in other forms, such as via the Internet or other wired or wireless telecommunication systems.

**Claims**

1. A wearable patch system, comprising:

    a wearable patch (12) comprising a sensor (14) configured to measure at least one sweat parameter; and
    a controller (16) configured to indicate the need to replace the patch,
    wherein the controller (16) is configured to:

        receive the at least one sweat parameter;
        compare the at least one sweat parameter to a first threshold;
        determine replacement information based on:

            the total amount of time during which the at least one sweat parameter exceeds the first threshold; and/or
            a value indicative of the at least one sweat parameter in respect of the total amount of time during which the at least one sweat parameter exceeds the first threshold; and

        output an indication that the wearable patch (12) needs replacing based on the replacement information.

2. The system of claim 1, wherein the replacement information comprises information that
    the total amount of time during which the at least one sweat parameter exceeds the first threshold exceeds a time threshold and/or
    the value indicative of the at least one sweat parameter in respect of the total amount of time during which the at least one sweat parameter exceeds the first threshold exceeds a value threshold.

3. The system of claim 1 or 2, wherein the value indicative of the at least one sweat parameter in respect of the total amount of time comprises an integration over time of a measured amount of a sweat component over said total amount of time or the rate of excretion over said total amount of time.

4. The system of any one of claims 1 to 3, wherein the patch (12) further comprises at least one further sensor (40) configured to monitor a further physiological parameter of the wearer.

5. The system of claim 4, wherein the at least one further sensor (40) is selected from sensors configured to monitor vital signs and sensors for biomarkers.

6. The system of any one of claims 1 to 5, wherein the at least one sweat parameter is selected from sweat volume, sweat pH, amount or rate of excretion of sodium, amount or rate of excretion of potassium, amount or rate of excretion of chloride, the amount or rate of excretion of at least one biomarker present in sweat and combinations thereof.

7. The system of any one of claims 1 to 6, wherein the sweat sensor (12) is configured to measure at least two sweat parameters.

8. The system of any one of claims 1 to 7, wherein the system (10) comprises:

an output device (42) configured to deliver the indication to a user of the system; and/or
a communications system (44) configured to communicate with an external output device for delivering the indication to a user of the system.

9. The system of claim 8, wherein the communications system (44) is a wireless communications system.

10. A method for providing an indication of the need to replace a wearable patch (12), the method comprising the steps of:

measuring (52) at least one sweat parameter of a person wearing the patch;
comparing (54) the at least one sweat parameter with a first threshold;
determining (56) replacement information based on:

the total amount of time during which the at least one sweat parameter exceeds the first threshold; and/or
a value indicative of the at least one sweat parameter in respect of the total amount of time during which the at least one sweat parameter exceeds the first threshold; and

providing (58) an indication that the wearable patch (12) needs replacing based on the replacement information.

11. The method of claim 10, wherein the replacement information comprises information that the total amount of time during which the at least one sweat parameter exceeds the first threshold exceeds a time threshold and/or the value indicative of the at least one sweat parameter in respect of the total amount of time during which the at least one sweat parameter exceeds the first threshold exceeds a value threshold.

12. The method of claim 10 or 11, wherein the at least one sweat parameter is selected from sweat volume, sweat pH, amount or rate of excretion of sodium, amount or rate of excretion of potassium, amount or rate of excretion of chloride, the amount or rate of excretion of at least one biomarker present in sweat and combinations thereof.

13. The method of any of claims 10 to 12, wherein the step of measuring (52) at least one sweat parameter comprises measuring at least two sweat parameters.

14. The method of any one of claims 10 to 13, wherein the value indicative of the at least one sweat parameter in respect of the total amount of time comprises an integration over time of a measured amount of a sweat component over said total amount of time or the rate of excretion over said total amount of time.

15. A computer program comprising computer program code which is adapted, when said program is run on a computer, to implement the method of any one of claims 10 to 14.

12

10

14

16

FIG. 1

FIG. 2

FIG. 3

FIG. 4

50

52 — measure sweat parameter

54 — compare sweat parameter with first threshold

56 — determine replacement information

58 — provide indication to replace patch

FIG. 5

**Europäisches Patentamt**
**European Patent Office**
**Office européen des brevets**

# EUROPEAN SEARCH REPORT

Application Number

EP 20 20 9475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2017/056650 A1 (COHEN ROBERT M [US] ET AL) 2 March 2017 (2017-03-02)<br>* paragraphs [0136], [0179] - [0226]; figures 1, 3, 5A-5D, 11A-C *<br>----- | 1-15 | INV.<br>A61B5/145<br>A61B5/00 |

TECHNICAL FIELDS
SEARCHED      (IPC)

A61B

The present search report has been drawn up for all claims

| Place of search | Date of completion of the search | Examiner |
|---|---|---|
| Berlin | 17 May 2021 | Dydenko, Igor |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 20 9475

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2021

| Patent document cited in search report | Publication date | Patent family member(s) | Publication date |
|---|---|---|---|
| US 2017056650 A1 | 02-03-2017 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170056650 A1